Europäisches Patentamt

European Patent Office

Office européen des brevets

(11) Publication number: **0 229 462 B1**

## EUROPEAN PATENT SPECIFICATION

(45) Date of publication of patent specification: **22.01.92**  (51) Int. Cl.⁵: **C07C 2/12**, C07C 2/26

(21) Application number: **86308655.9**

(22) Date of filing: **06.11.86**

(54) Process for the manufacture of high viscosity lubricating oils.

(30) Priority: **13.12.85 US 808705**

(43) Date of publication of application:
**22.07.87 Bulletin 87/30**

(45) Publication of the grant of the patent:
**22.01.92 Bulletin 92/04**

(84) Designated Contracting States:
**BE DE ES FR GB IT NL**

(56) References cited:
**US-A- 4 520 221**

**INDUSTRIAL & ENGINEERING CHEMISTRY PRODUCT RESEARCH AND DEVELOPMENT, vol. 19, no. 1, March 1980, American Chemical Society, Washington, D.C., US; J.A. BRENNAN "Wide-temperature range synthetic hydrocarbon fluids"**

**CHEMICAL ABSTRACTS, vol. 99, no. 18, 31st October 1983, page 150, column 2, abstract no. 142646h in combination with Chemical Substance Index, page 4853C2, column 2, lines 61-64, Columbus, Ohio, US; A. ONOPCHENKO et al.: "The boron fluoride-catalyzed oligomerization of alkenes - structures, mechanisms and properties"**

(73) Proprietor: **MOBIL OIL CORPORATION**
**150 East 42nd Street**
**New York New York 10017(US)**

(72) Inventor: **Chester, Arthur Warren**
**517 Country Club Drive**
**Cherry Hill New Jersey 08003(US)**
Inventor: **Garwood, William Everett**
**125 Warwick Road**
**Haddonfield New Jersey 08033(US)**
Inventor: **Tabak, Samuel Allen**
**204 East Pine Street**
**Wenonah New Jersey 08090(US)**

(74) Representative: **West, Alan Harry**
**Mobil Court 3 Clements Inn**
**London WC2A 2EB(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to the manufacture of lubricating oils by the oligomerization of olefins.

Recent work in the field of olefin upgrading has resulted in catalytic processes for converting lower olefins into heavier hydrocarbons. Heavy distillate and lubricant range hydrocarbons can be synthesized over catalysts comprising ZSM-5 type zeolites at elevated temperatures and pressures, the product having substantially linear molecular conformations due to the ellipsoidal shape selectively of certain medium pore zeolites.

Conversion of olefins into gasoline and/or distillate products is described in U.S. Patents 3,960,978 and 4,021,502, gaseous olefins in the range of ethylene to pentene, either alone or in admixture with paraffins, being converted into olefinic gasoline blending stock by contacting the olefins with a catalyst of a ZSM-5 type zeolite. U.S. Patents 4,150,062, 4,211,640 and 4,227,992 describe the operating conditions of the Mobil Olefin to Gasoline/Distillate (MOGD) process for the selective conversion of $C_3$+ olefins into mainly aliphatic hydrocarbons.

In the catalytic conversion of olefins into heavier hydrocarbons by oligomerization over a medium pore, shape selective, acid, crystalline zeolite, such as ZSM-5, process conditions can be varied to favor the formation of hydrocarbons of various molecular weights. At moderate temperatures and relatively high pressures, the conditions favor $C_{10}$+ aliphatic distallate products. Lower olefinic feedstocks containing $C_2$-$C_8$ alkenes may be converted by such a procedure. However, such distillate mode conditions do not convert a major fraction of ethylene. A typical reactive feedstock consists essentially of $C_3$-$C_6$ mono-olefins, with varying amounts of nonreactive paraffins and the like being acceptable components.

The above-described shape-selective oligomerization, as it applies to the conversion of $C_2$-$C_{10}$ olefins over ZSM-5, is known to produce higher olefins up to $C_{30}$ and higher. As reported by Garwood in Intrazeolite Chemistry 23,(Amer. Chem. Soc., 1983), reaction conditions favoring higher molecular weight product are low temperatures (200-260°C), elevated pressures (about 2000 kPa or greater), and long contact times (less than 1 WHSV). The reaction under these conditions proceeds through the acid-catalyzed steps of (1) oligomerization, (2) isomerization-cracking to a mixture of intermediate carbon number olefins, and (3) interpolymerization to give a continuous boiling product containing all carbon numbers. The channel systems of ZSM-5 type zeolites impose shape-selective constraints on the configuration of the larger molecules, accounting for the differences from other catalysts.

The desired oligomerization-polymerization products include $C_{10}$+ substantially linear aliphatic hydrocarbons. The ZSM-5 zeolite catalytic path for propylene, for example, provides a long chain with approximately one alkyl (for example, methyl) substituent per 5 or more carbon atoms in the main chain. The lubricant range final product can be depicted as a typical linear molecule having a sparingly-substituted (saturated) long carbon chain.

The final molecular conformation is influenced by the pore structure of the zeolite. For the higher carbon numbers, the structure is primarily a methyl-branched straight olefinic chain, with the maximum cross section of the chain limited by the pore size constraints of ZSM-5. Although emphasis is placed on the normal 1-alkenes as feed stocks, other lower olefins such as 2-butene or isobutylene, are readily employed as starting materials due to rapid isomerization over the acidic zeolite. Other mixed olefin streams can also be employed such as FCC off gas or Fischer Tropsch

The viscosity index of a hydrocarbon lube oil is related to its molecular conformation. Extensive branching in a molecule usually results in a low viscosity index. It is believed that two modes of oligomerization/polymerization of olefins can take place over acidic zeolites such as HZSM-5. One reaction sequence takes place at shape constrained Bronsted acid sites inside the channels or pores, producing essentially linear materials. The other reaction sequence occurs on the outer surface, producing highly branched material. By decreasing the surface acid activity (surface alpha value) of such zeolites, fewer highly branched products with low VI are obtained. Thus, U.S. Patent 4,520,221 is concerned with the conversion of olefins into a 340°C+ lube fraction by contacts the olefins over ZSM-5 or related zeolite catalyst at elevated temperatures and pressured, the catalyst having been treated with a bulky alkylpyridine or other basic compound whereby the surface activity or acidity of the zeolite is removed or substantially eliminated.

Also, U.S. Patent 4,547,613 describes forming a lube oil by contacting olefins with ZSM-5 or related zeolite which has been conditioned by previous contact with a light olefin.

Other patents which describe the conversion of olefins over zeolites such as ZSM-5 into higher boiling products such as 340°C+ lube fractions include U.S. Patents 4,517,399 (contacting the olefins with ZSM-5 or related zeolite having a crystalline size greater than 2 microns); 4,126,644 (conversion of a $C_5$-200°C liquid fraction from a Fischer-Tropsch synthesis, predominately $C_5$-$C_{10}$ olefins); and 3,322,848 (manufacture

2

of high VI, low pour point lube oils from $C_{10}$-$C_{18}$ normal alpha olefins over crystalline aluminosilicates other than those related to ZSM-5).

Another catalyst useful in forming lubricants from the polymerization of olefins having from 5 to 14 carbon atoms per molecule, is ditertiary alkyl peroxide as described in U.S. Patent 2,937,129. Such catalysts are also referred to in "Wide-Temperature Range Synthetic Hydrocarbon Fluids" (Brennan, Ind. Eng. Chem. Prod. Res. Dev.,1980, 19, pp 2-6), where the oligomer selectivity of ditertiary butyl peroxide in 1-decene oligomerisation is disclosed.

U.S. Patent 4,568,786 describes a two stage olefin to lure conversion process wherein a lower olefin feed is oligomerized in a primary stage with a medium pore shape-selective siliceous zeolite catalyst having acid cracking activity, and a constraint index of about 1 to 12 and wherein the zeolite surface is rendered substantially inactive for acidic reactions chemisorption of a surface deactivating agent; and at least a portion of the primary stage effluent is converted in a secondary reactor stage with an acid catalyst (BF$_3$) to produce a high viscosity index lubricant range hydrocarbon.

One limitation of olefin oligomerization sub as the MOGD process for producing lubes has been that higher viscosity lubes are not easily obtainable. The present invention seeks to overcome that limitation and to produce high viscosity lubes which have a low pour point and high viscosity index.

In accordance with the invention, there is provided a process for producing a high viscosity lubricant which comprises contacting a feedstock comprising one or more lower olefins with an oligomerisation catalyst comprising a medium-pore siliceous zeolite at a temperature of 175 to 375°C, a pressure of 790 to 14,000 kPa and a LHSV of 0.2 to 10, separating from the effluent from said contacting a 165°C + hydrocarbon fraction and contacting said fraction with a ditertiary alkyl peroxide catalyst at a temperature of 100 to 200°C to yield a product comprising a 340°C + fraction composed of high viscosity lubricant range hydrocarbons. In accordance with this process, a lower olefin feed is oligomerized at moderate temperature and elevated pressure in a first stage, and after the first stage effluent has been separated to obtain a heavy fraction rich in substantially linear $C_{10}$ + intermediate olefins (165°C +), the process is completed by contacting the heavy fraction in a second stage with a ditertiary alkyl peroxide catalyst to upgrade the heavy fraction to a high viscosity index lubricant range hydrocarbon. This technique is advantageous for producing high viscosity $C_{20}$ + heavy hydrocarbons comprising lubricant range compounds (340°C +) having viscosity indices and pour points about the same as those obtained MOGD lube oils of lower viscosity.

Generally, the first stage of the process involves oligomerization of an inexpensive lower olefin, for example propylene, at a moderate temperature over a shape-selective, crystalline aluminosilicate zeolite. The second stage involves further oligomerization/interpolymerization of the product (or a fraction of the product) from the first stage over a second and different catalyst at about 100-200°C.

In the first stage of the process, light olefins, for example $C_2$-$C_6$, are converted into distillate boiling range hydrocarbons by the MOGD process.

The oligomerization catalysts preferred for use in MOGD include those comprising the medium pore (about 5-7 Angstroms), shape-selective, crystalline aluminosilicate zeolites having a silica to alumina ratio of at least 12, a constraint index of 1-12 and acid cracking activity of 50-200. Representative of the shape selective zeolites are ZSM-5, ZSM-11, ZSM-12, ZSM-22, ZSM-23, ZSM-35, ZSM-38, and ZSM-48. ZSM-5 is described in U.S. Patents 3,702,886 and Reissue 29,948. Other suitable zeolites are described in U.S. Patents 3,709,979 (ZSM-11); 3,832,449 (ZSM-12); 4,076,979; 4,076,842 (ZSM-23); 4,016,245 (ZSM-35); 4,046,339 (ZSM-38); and 4,375,573 (ZSM-48).

A further useful catalyst is a medium pore, shape-selective, crystalline aluminosilicate zeolite as described above containing at least one Group VIII metal, for example Ni-ZSM-5. This catalyst is able to convert ethylene at moderate temperatures. Similarly, U.S. Patents 4,542,251 and 4,517,396 describe processes for oligomerizing olefins over siliceous crystalline molecular sieve catalysts which contain nickel.

The catalyst for oligomerization of the olefinic feed can be composited with a suitable binder such as alumina and shaped in the form of cylindrical extrudates of about 1-5 millimeters diameter for use in fixed bed operation or particle size for use in fluid bed operation. Other pentasil catalysts which may be employed for coverting lower olefins include those comprising a variety of medium pore (~5 to 9A) siliceous materials such as borosilicates, ferrosilicates, and/or aluminosilicates described in U.S. Patents 4,4l4,423, 4,4l7,O86, and 4,5l7,396.

To reduce excessive branching in the lube molecule catalysts of low surface activity can be used. Such low surface active catalysts can be obtained by using medium pore zeolites of small crystal size that have been deactivated by basic compounds, examples of which are amines, phosphines, phenols, polynuclear hydrocarbons, cationic dyes and others. These compounds all must have a minimum cross sectional diameter of 5A. Examples of suitable amines include monoamines, diamines, triamines, aliphatic and

EP 0 229 462 B1

aromatic cyclic amines and heterocyclic amines, porphines, phthalocyanines, I,IO-phenanthroline, 4,7-diphenyl-I, IO-phenanthroline, 3,4,7,8-tetramethyl-I, IO-phenanthroline, 5,6-benzoquinoline, $2,2':6',2''$-terpyridine, 2,4,6-tri(2-pyridyl)-S-triazine and 2,3-cyclododecenopyridine. Examples of phosphines include triphenylphospine and I,2-bis(diphenylphosphine)ethane. Suitable phenols are, for example, di-t-butylphenol, alkylated naphthol and 2,4,6-trimethylphenol. Polynuclear hydrocarbons include substances such as pyrene and phenanthrene. Cationic dyes include thionine, methylene blue and triphenylmethane dyes, such as malachite green and crystal violet. Another surface modification technique is deactivation by treating with metal compounds. Suitable metal compounds are magnesium acetate, metal-porphines, such as hemin or iron (III) porphine chloride, cobalticinium chloride $(C_5H_5)_2CoCl$, and titanocene dichloride (biscyclopentadienyl titanium dichloride), large complex cations such as $[Co(NH_2R)_6]^{2+}$ where R = H or alkyl, $[Pt(NH_2R)_4]^{2+}$ where R = alkyl, $[co(en)_3]^{3+}$ where en = ethylenediamine and manganese (III) meso-tetraphenylporphine.

The zeolites may also be treated with organic silicon compounds, as described in U.S. Patents 4,100,215 and 4,002,697 to impart the desired degree of surface deactivation while being essentially free of carbonaceous deposits. Such treatment involves contacting the zeolite with a silane surface modifying agent capable of deactivating catalytic (acidic) sites located on the external surface of the zeolite by chemisorption. Those amines with an effective cross section larger than about 5 Angstroms are suitable especially substituted quinolines, heterocyclic amines and alkyl substituted pyridines such as 2,4 or 2,6-di-alkyl pyridines. Preferred are bulky, sterically-hindered di-ortho-alkyl pyridines, such as 2,6-di-tertiary-butylpyridine. The lower molecular weight $C_6$-$C_{20}$ intermediate materials formed over the modified catalysts are relatively linear olefins.

More particularly, since the first stage MOGD is operated to obtain a greater proportion of distillate fuels, that is a $C_{10}+$ fraction boiling at 165°C and above, the MOGD reaction is generally run at a moderate temperature of 190 to 320°C and relatively high pressure of about 2850 to 14,000 kPa. The MOGD reactor can be of the fixed, moving or fluidized bed type.

The effluent mixture from the first stage MOGD is separated into a high boiling product as a liquid rich in $C_{10}+$ hydrocarbons, and a vapor component including light gas and lower hydrocarbons, such as $C_1$ to $C_9$ aliphatics. The MOGD $C_{10}+$ liquids are then converted into high viscosity index, low pour point lubes by conversion over a ditertiary alkyl peroxide catalyst.

In general, the synthetic lubricants are produced in the second stage of the process by contacting the MOGD $C_{10}+$ liquids at a temperature of about 100 to about 200°C, with a ditertiary-alkyl peroxide catalyst.

The second stage catalyst used to produce the polymerized olefin synthetic lubricants according to the invention are ditertiary-alkyl peroxides. These peroxides can be represented by the formula, $ROOR'$, in which R and $R'$ are the same or different, tertiary alkyl groups. Preferably, the tertiary alkyl groups are lower tertiary alkyl groups. Examples are ditertiary-butyl peroxide, ditertiary-amyl peroxide, and tertiary-butyl tertiary-amyl peroxide.

The amount of peroxide catalyst employed in the process determines the viscosity of the polyolefin synthetic lubricant product. In general, the more viscous lubricants are obtained when larger amounts of catalyst are used. The amount of ditertiary-alkyl peroxide catalyst employed will generally be from about O.OI to about O.3 mole per mole of MOGD liquid reactant.

The ditertiary-alkyl peroxide catalyst can be added to the first stage MOGD reactant in a single addition. It is preferred, however, to add the catalyst in two or more portions at intervals of several minutes to several hours. A feasible procedure is to add the catalyst gradually to the reaction mixture throughout the course of the reaction.

The temperature employed in the second stage of the process is the activation temperature of the catalyst. The ditertiary-alkyl peroxides are activated at temperatures varying from about IOO to about 2OO°C, depending upon the particular peroxide selected. The pressure employed depends upon the temperature used and upon the reactant. Ordinarily, a pressure sufficient to maintain the reactants substantially in the liquid phase is sufficient. The time of reaction depends, of course, upon the temperature employed, the nature of the reactants, and to some extent the pressure. In general, the higher the reaction temperature, the shorter the reaction time required. The criterion used is the time required, at a given temperature, to effect condensation and to assure substantially complete utilization of the catalyst. In general, the time of reaction will vary between about one hour and about 6 hours.

At the temperatures required for the second stage conversion, some of the ditertiary-alkyl peroxide catalysts together with alcohol may tend to distill from the reaction zone. This material can be gathered in a suitable trap or receiver and recycled to the reaction zone. It is preferable, not necessary, to wash this material with water to remove alcohol prior to recycling.

Advantageously, the second stage effluent liquid stream is fractionated to provide a product stream

4

comprising a major amount of $C_{10}$-$C_{20}$ distillate and $C_{20}$-$C_{60}$ aliphatic hydrocarbons.

This olefinic product may then be hydrotreated in a separate process step to provide a paraffinic lubricant and/or heavy distillate product. Details of a mild hydrogenation treatment may be obtained from U.S. Patent 4,211,640, typically using Co or Ni with W/Mo and/or noble metals. The hydrotreated stream may be further fractionated to yield refined high grade lubricants of outstanding quality.

As mentioned above, the 340°C+ product of the invention is a lube oil of outstanding properties. The lubricants have a viscosity as high as 280 mm²/s at 38°C and 17 mm²/s at 99°C. Higher viscosities may be obtained by increasing the amount of peroxide. Viscosity indices and pour points are about the same as those obtained in MOGD-L when compared at lower viscosities.

The following Examples illustrate the invention.

EXAMPLE I

The MOGD liquid was prepared by processing a mixed olefin of propane/propylene/butane/butylene over HZSM-5 extrudate catalyst in a pilot unit run at 1 LHSV (olefin), 5600 kPa, 2/1 recycle of 21-230°C product/fresh olefin, 250-255°C over a 12-22 day period. The 165°C+ product, 84 wt.% yield based on fresh feed, had the following properties:

```
Gravity, °API              44.7
      Specific              0.8031
Hydrogen, wt.%             14.32
Bromine No.                60.5
Aniline Point              175
Distillation, °C, ASTM  D-2887
       0.5%                157
        10                 226
        20                 239
        30                 249
        40                 259
        50                 271
        60                 284
        70                 302
        80                 322
        90                 351
        95                 373
        99.5               431
```

300 g of the liquid was placed in a 1 liter round bottom flask equipped with a stirrer, thermometer, water condenser, and dropping burette. The flask was heated to 150°C, and 12 g (15.6 ml) ditertiary butyl peroxide (DTBP) added dropwise from the burette over a 1 hour period. The temperature was held at 150°C for an additional 3 hours, then raised to 186°C in the next 2 hours. The contents were then cooled to room temperature and were analyzed as follows:

```
Gravity, °API              43.7
    Specific               0.8076
Distillation, °C,
     1%                    43
    10                     219
    20                     237
    30                     249
    40                     262
    50                     277
    60                     297
    70                     322
    80                     357
    90                     410
    95                     445
    99.5                   509
```

The liquid product was distilled first at atmospheric pressure to remove lighter boiling material, then under reduced pressure to separate a 2l wt.% ~340°C + bottoms fraction having the following properties:

```
Gravity, °API              35.3
    Specific               0.8483
Pour Point, °C             < -54
KV @ 40°C, mm²/s           35.90
KV @ 100°C, mm²/s          5.47
(SUS @ 100°F               186)
Viscosity Index            81.6
```

EXAMPLE 2

The MOGD liquid of Example I was distilled to give 89 wt.% overhead boiling below 340°C, and ll wt.% 340°C + bottoms having the following properties:

```
Gravity, °API              38.2
    Specific               0.8338
Pour Point, °C             -51
KV @ 40°C, mm²/s           14.29
KV @ 100°C, mm²/s          3.225
(SUS @ 100°F               79)
Viscosity Index            84.0
```

This bottoms material was a part of the charge to Example I. However, it can be seen that the DTBP polymerized product of Example I has about the same viscosity index and pour point, and is much more viscous (l86 vs 79 SUS @ l00°F).

EXAMPLE 3

3OO g of the overhead material boiling below 340°C from Example 2 was polymerized with DTBP in the same manner as in Example I, except that the procedure was repeated twice more, using a total of 36 g (46.8 ml) DTBP. The liquid product was analyzed as follows:

6

```
Gravity, °API                41.7
    Specific                  0.8170
Distillation, °C,
       1%                     50
      10                      226
      20                      244
      30                      258
      40                      275
      50                      299
      60                      329
      70                      376
      80                      414
      90                      456
      95                      484
      99.5                    532
```

The liquid product was distilled yielding a 40 wt.% 340°C+ bottoms lube having the following properties.

```
Gravity, °API                34.8
      Specific                0.8509
Pour Point, °C              -48
KV @ 40°C, mm²/s            48.79
KV @ 100°C, mm²/s            6.51
(SUS @ 100°F               253)
Viscosity Index             77.1
```

The ~340°C+ bottoms lube was distilled further at 4 Pa pressure, to give 51% ~425°C+ bottoms (20 wt.% based on original charge) having the following properties:

```
Gravity, °API                32.1
      Specific                0.8649
Pour Point, °C              -9.5
KV @ 40°C, mm²/s           243.4
KV @ 100°C, mm²/s           16.59
(SUS @ 100°F              1309*)
Viscosity Index             61.3

        (*86.6 SUS @ 210°F)
```

This Example demonstrates that very viscous lubes can be produced by increasing the amount of DTBP and distilling beyond the normal 340°C cut point.

EXAMPLE 4

The liquid charge for this Example was prepared by processing a propylene/butylene stream (propane/propylene/butane/butylene, 10.5/26.8/27/35.1) over HZSM-5 extrudate in a pilot plant run at ~0.5 LHSV, 10450 kPa, with recycle of 21 to ~315°C liquid and temperature 200-235°C. The liquid product was distilled to give 57 wt.% 165-340°C overhead, and 39 wt.% 340°C+ MOGD lube product having the following properties:

```
Gravity, °API              35.4
    Specific               0.8478
Pour Point, °C             <-54
KV @ 40°C, mm2/s           17.53
KV @ 100°C, mm2/s           3.615
(SUS @ 100°F               94)
Viscosity Index            78.7
```

The l65-340°C liquid had the following properties:

```
Gravity, °API              42.2
    Specific               0.8146
Bromine No.                46.2
Aniline Point              171
Distillation, °C,
    0.5%                   140
    5                      184
    10                     200
    20                     229
    30                     257
    40                     283
    50                     301
    60                     312
    70                     321
    80                     328
    90                     337
    95                     343
    95.5                   368
```

300 g of this liquid was polymerized with DTBP in the same manner as in Example 3, and then distilled to give 4l wt.% 340°C+ bottoms product having the following properties:

```
Gravity, °API              32.1
    Specific               0.8649
Pour Point, °C             -43
KV @ 40°C, mm2/s           64.05
KV @ 100°C, mm2/s           7.465
(SUS @ 100°F               335)
Viscosity Index            69.7
```

EXAMPLE 5

The 340°C+ lube product from Example 4 was hydrogenated using a nickel catalyst. 75 g of the lube was diluted with 200 ml cyclohexane and charged along with 2.3 g of the catalyst to a 2 liter stirring autoclave. The autoclave was purged three times with 3550 kPa nitrogen, then three times with hydrogen, then finally pressured to 2860 kPa hydrogen and heated at l75°C for l.5 hours. The contents were cooled and the cyclohexane distilled off, leaving a bottoms product with the following properties:

8

```
Gravity, °API              32.9
    Specific                  0.8607
Pour Point, °C            -46
KV @ 40°C, mm²/s          63.69
KV @ 100°C, mm²/s          7.38
(SUS @ 100°F             333)
Viscosity Index           67.2
```

The Example shows that hydrogenation has little effect on the physical properties of the lube.

EXAMPLE 6

The liquid charge for this Example was prepared by processing a propylene/butylene stream over a 2,6 ditertiary butyl pyridine pretreated HZSM-5 catalyst. The pretreating was by wetting the catalyst with a mixture of 2,6 DTBP in n-hexane followed by evaporation of the hexane. Reaction conditions were 7000 kPa, 0.5 LHSV (olefin), ~205°C over a five day period. The liquid product was distilled to give 55.8 wt.% 165-340°C overhead, and 10.7 wt.% 340°C+ lube product having the following properties:

```
Gravity, °API              34.3
    Specific                  0.8534
Pour Point, °C          < -18
KV @ 40°C, mm²/s          19.25
KV @ 100°C, mm²/s          4.02
(SUS @ 100°F             102)
Viscosity Index          125.0
```

250 g of the 165-340°C overhead liquid was polymerized with DTBP (30 g, 39 ml) in the same manner as in Example 3, and then distilled to give 24 wt.% 340°C+ bottoms product having the following properties:

```
Gravity, °API              36.0
    Specific                  0.8448
Pour Point, °C          < -54
KV @ 40°C, mm²/s          40.83
KV @ 100°C, mm²/s          5.98
(SUS @ 100°F             211)
Viscosity Index           85.6
```

The viscosity index is higher than that of the 340°C+ lubes produced with DTBP in the previous examples. This is believed to be due to a more linear mixture of olefinic molecules in the 165-340°C charge, compared to the previous examples where secondary non-shape selective surface reactions degrade (isomerize and crack) the primary olefin mixture.

EXAMPLE 7

Isomers of C₈ olefins were oligomerized in the presence of ditertiary butyl peroxide catalyst. The reactions were done at 150°C, using 25.8 wt.% peroxide on olefin.

9

| Olefin Charge | Octene-1 | Octene-2 | 2-Ethyl-Hexene-1 |
|---|---|---|---|
| Oil Yield, Wt.% | 20.9 | 2.6 | 1.1 |
| Pour Point, °C | < -34 | < -34 | -26 |
| K.V. @ 38°C, $mm^2/s$ | 111.9 | 55.34 | 190.4 |
| K.V. @ 99°C, $mm^2/s$ | 13.53 | 7.06 | 11.95 |
| Viscosity Index | 120 | 91 | 29 |

The data show that both an internal double bond and ethyl branching next to a terminal bond limit lube yields severely, and in addition the ethyl branching decreases viscosity index markedly.

EXAMPLE 8

The charge stock in this example was a blend of equal weights of Shell Chemical straight chain l-olefins, $C_{10}$, $C_{12}$, $C_{14}$, $C_{16}$, $C_{18}$, $C_{20}$. The boiling range of the blend covered essentially the same 165-340°C range of Example 3. Results are as follows:

| Run No. | 1 | 2 |
|---|---|---|
| Peroxide, wt.% on olefin | 4[1] | 12[2] |
| 340°C+ Lube yield, wt.% | 60.6 | 94.7 |
| Gravity, °API | 37.3 | 34.2 |
| Specific | 0.8383 | 0.8545 |
| Pour Point, °C | +10 | - |
| K.V. @ 40°C, $mm^2/s$ | 66.92 | 476.0 |
| K.V. @ 100°C, $mm^2/s$ | 12.40 | 54.06 |
| (SUS @ 100°F | 338 | 2460) |
| (SUS @ 210°F | 69 | 260) |
| Viscosity Index | 187 | 180 |

(1) 300 g olefin blend, 12 g DTBP added as in Example 1
(2) 300 g olefin blend, 36 g DTBP added as in Example 3

Compared to the 165-340°C MOGD liquid charge of Example 3, the l-olefin blend gives a much higher yield of very viscous, high viscosity index lube, but also a much higher pour point, too high for use as a lubricant.

**Claims**

1. A process for producing a high viscosity lubricant which comprises contacting a feedstock comprising one or more lower olefins with an oligomerisation catalyst comprising a medium-pore siliceous zeolite at a temperature of 175 to 375°C, a pressure of 790 to 14,000 kPa and a LHSV of 0.2 to 10, separating from the effluent from said contacting a 165°C+ hydrocarbon fraction and contacting said fraction with a ditertiary alkyl peroxide catalyst at a temperature of 100 to 200°C to yield a product comprising a 340°C+ fraction composed of high viscosity lubricant range hydrocarbons.

2. A process according to claim 1, wherein the ologomerization catalyst comprises a crystalline aluminosilicate zeolite having a constraint index of 1 to 12.

3. A process according to claim 2, wherein the zeolite is ZSM-5.

4. A process according to claim 2 or claim 3, wherein the surface of the zeolite has been rendered substantially inactive for acid reactions by chemisorption of a surface deactivating agent.

**5.** A process according to claim 4, wherein the surface deactivating agent is a dialkylpyridine.

**6.** A process according to any one of claims 1 to 5, wherein the lower olefin feedstock comprises a mixed stream of $C_2$-$C_6$ olefins.

**7.** A process according to any one of claims 1 to 6, wherein oligomerization is carried out at a temperature of 190 to 320°C and at a pressure of 2850 to 14,000 kPa.

**8.** A process according to any preceding claim wherein from 0.01 to 0.3 mole of ditertiary alkyl peroxide catalyst is employed per mole of said 165°C+ hydrocarbon fraction.

**9.** A process according to claim 8, wherein the peroxide catalyst is ditertiary butylperoxide.

**Revendications**

**1.** Un procédé de production d'un lubrifiant de viscosité élevée qui consiste à mettre en contact une charge comprenant une ou plusieurs oléfines inférieures avec un catalyseur d'oligomérisation comprenant une zéolite siliceuse à pores moyens, cette oligomérisation étant effectuée à une température de 175 à 375°C, une pression de 790 à 14 000 kPa et une VSHL (vitesse spatiale horaire liquide) de 0,2 à 10, à séparer de l'effluent résultant dudit contact une fraction hydrocarbonée à point d'ébullition de 165°C+ et à mettre au contact ladite fraction avec un catalyseur à base de ditertioalkylperoxyde à une température comprise entre 100 et 200°C pour conduire à l'obtention d'un produit présentant une fraction bouillant à 340°C+ formée d'un hydrocarbure de la gamme des lubrifiants présentant une viscosité élevée.

**2.** Un procédé selon la revendication 1, dans lequel le catalyseur d'oligomérisation est formé d'une zéolite à base d'aluminosilicate cristallin présentant un indice de contrainte compris entre 1 et 12.

**3.** Un procédé selon la revendication 2, dans lequel la zéolite consiste en ZSM-5.

**4.** Un procédé selon la revendication 2 ou la revendication 3, dans lequel la surface de la zéolite a été rendue pratiquement inactive pour des réactions acides par chémisorption d'un agent de désactivation de surface.

**5.** Un procédé selon la revendication 4, dans lequel l'agent de désactivation de surface est une dialkylpyridine.

**6.** Un procédé selon une quelconque des revendications 1 à 5, dans lequel la charge oléfinique inférieure consiste en un courant formé d'un mélange d'oléfines en $C_2$ à $C_6$.

**7.** Un procédé selon une quelconque des revendications 1 à 6, dans lequel l'oligomérisation est effectuée à une température comprise entre 190 et 320°C sous une pression de 2 850 à 14 000 kPa.

**8.** Un procédé selon une quelconque des revendications précédentes, dans lequel on emploie de 0,01 à 0,3 mole de catalyseur à base de ditertio-alkylperoxyde par mole de la fraction hydrocarbonée bouillant à 165°C+.

**9.** Un procédé selon la revendication 8, dans lequel le catalyseur peroxydique consiste en ditertio-butylperoxyde.

**Patentansprüche**

**1.** Verfahren zur Herstellung eines hochviskosen Schmiermittels, das den Kontakt eines Ausgangsmaterials, das ein oder mehrere niedere Olefine umfaßt, mit einem Oligomerisierungs-Katalysator, der einen siliciumhaltigen Zeolith mit mittleren Poren umfaßt, bei einer Temperatur von 175° - 375°C, einem Druck von 790 - 14.000 kPa und einer LHSV von 0,2 - 10, die Abtrennung einer 165°C+-Kohlenwasserstoff-Fraktion vom Abfluß aus diesem Kontakt und den Kontakt dieser Fraktion mit einem ditertiären Alkylperoxid-Katalysator bei einer Temperatur von 100° - 200°C umfaßt, um ein Produkt zu erzeugen, das eine 340°C$^+$-Fraktion umfaßt, die aus hochvoskosen Kohlenwasserstoffen in Schmiermittelbereich zusammengesetzt ist.

2. Verfahren nach Anspruch 1, worin der Oligomerisierungs-Katalysator einen kristallinen Aluminosilicatzeolith mit einem Zwangsindex von 1 - 12 umfaßt.

3. Verfahren nach Anspruch 2, worin der Zeolith ZSM-5 ist.

4. Verfahren nach Anspruch 2 oder 3, worin die Oberfläche des Zeoliths durch aktivierte Sorption eines die Oberfläche deaktivierenden Mittels für saure Reaktionen im wesentlichen inaktiv gemacht wurde.

5. Verfahren nach Anspruch 4, worin das die Oberfläche deaktivierende Mittel Dialkylpyridin ist.

6. Verfahren nach einem der Ansprüche 1 - 5, worin das niedere olefinische Ausgangsmaterial einen gemischten Strom von $C_2$-$C_6$-Olefinen umfaßt.

7. Verfahren nach einem der Ansprüche 1 - 6, worin die Oligomerisierung bei einer Temperatur von 190° - 320°C und bei einem Druck von 2.850 - 14.000 kPa durchgeführt wird.

8. Verfahren nach einem der vorstehenden Ansprüche, worin von 0,01 - 0,3 Mol des ditertiären Alkylperoxid-Katalysators pro Mol der 165°C$^+$-Kohlenwasserstoff-Fraktion eingesetzt werden.

9. Verfahren nach Anspruch 8, worin der Peroxidkatalysator ditertiäres Butylperoxid ist.